# EUROPEAN PATENT APPLICATION

(11) **EP 1 873 205 A1**
(43) Date of publication of application: **02.01.2008**
(21) Application number: 06300583.9
(22) Date of filing: 12.06.2006
(51) Int. Cl.: C08L 33/26, C08L 71/02, G01N 33/543, B01J 19/00

(54) **Thermo-responsive blends and uses thereof**

(71) Applicant: Corning Incorporated, Corning, NY 14831 (US)
(72) Inventor: Carre, Alain, 77820, Le Chatelet en Brie (FR); Dufour, Bruno, 77210, Avon (FR); Henry, David, 91150, Morigny-Champigny (FR); Waku-Nsimba, Jean, 77940, La Brosse-Montceaux (FR)
(74) Representative: Marchant, James Ian

(57) **Abstract**

Described herein are thermo-responsive blends. Also described herein are articles, substrates, and kits composed of thermo-responsive blends for immobilizing cells or tissue and methods of use thereof.

## Description

### BACKGROUND

The ability to proliferate and differentiate cell growth *in vivo* has numerous applications. Cell growth *in vivo* occurs in the extracellular matrix (ECM), which is a three-dimensional environment. A two-dimensional surface such as a Petri dish surface for example, is not representative of cells growing *"in vivo."* One function of the three dimensional environment is to direct cell behavior such as migration, proliferation, differentiation, maintenance of the phenotypes and apoptosis by facilitating sensing, and responding to the environment via cell-matrix and cell-cell communications. Therefore, a material having proper porosity, large surface area, and well interconnected pores is desirable for culturing cells. In particular, to achieve efficient cell culturing that is comparable to *in vivo* cell growth, it is desirable that the material permit the permeation of cells through the entire material.

Cell culturing is generally carried out by seeding the substrate with cells in the presence of a biological medium. After adhesion and proliferation, the cultured cells are typically harvested using invasive methods, such as mechanical scraping, sonication, chemical or enzymatic treatment, or a combination thereof. Mechanical scraping can damage the cells, and it is not convenient in confined spaces such as small diameter wells, or other 3D substrates. Chemical (e.g., EDTA) or biological (e.g., trypsin) treatments may harm the cells and present a risk of introducing impurities. For example, trypsin is well-known for its of cell function. Moreover, very adherent cells are difficult to harvest using these and require a long, damaging exposure.

Therefore, there is a need for an easy, noninvasive, and effective way of harvesting viable cells from culture substrates. Non-invasive, non-destructive alternate methods of cell culture harvesting would be a significant improvement because it would minimize handling, minimize additives and contamination, allow for cells to be harvested intact, and allow for efficient recovery of cells and products from cells, particularly membrane bound products. Described herein are substrates that facilitate the immobilization and subsequent growth of cells and tissues. Additionally, the substrates described herein permit the facile release and isolation of cells and tissues cultured on the substrates.

### SUMMARY

In accordance with the purposes of the disclosed materials, compounds, compositions, articles, devices, and methods, as embodied and broadly described herein are thermo-responsive blends. Also described herein are substrates, articles, and kits composed of the thermo-responsive blends for immobilizing cells or tissue and methods of use thereof. Additional advantages will be set forth in part in the description that follows, and in part will be obvious from the description, or may be learned by practice of the aspects described below. The advantages described below will be realized and attained by means of the elements and combinations particularly pointed out in the appended claims. It is to be understood that both the foregoing general description and the following detailed description are exemplary and explanatory only and are not restrictive.

### BRIEF DESCRIPTION OF THE DRAWINGS

The accompanying figures, which are incorporated in and constitute a part of this specification, illustrate several aspects described below.
Figure 1 shows the water contact angle of polystyrene/polyNIPAAm blends at 45 and 20 °C, where the amount of polyNIPAAm in the blend is between 0 and 25 wt%.
Figure 2 shows the number of CHO cells released by successively lowering the temperature to 4 °C for 2 hours and exposing to trypsin for ten minutes using blends prepared via solution processing as cell culture substrates.
Figure 3 shows the number of CHO cells released by successively lowering the temperature to 4 °C for 2 hours and exposing to trypsin for ten minutes using blends prepared via extrusion/hot pressing as cell culture substrates.
Figure 4 shows the water contact angle of thick films composed of blends of PS+PPO-PEO-PS at two different temperatures prepared by evaporation of a solution in anisole.
Figure 5 shows the water contact angle at two different temperatures of discs composed of blends of PS+PPO-PEO-PS prepared by extrusion and hot press.
Figure 6 is a SEM picture (500 X magnification) showing the morphology of a fibrillar material produced from 5 wt % poly(N-isopropylacrylamide) and 95 wt% PMMA.
Figure 7 is a SEM picture (800 X magnification) showing the morphology of a fibrillar material produced from 5 wt % poly(N-isopropylacrylamide) and 95 wt% PMMA.

### DETAILED DESCRIPTION

The materials, compounds, compositions, articles, devices, and methods described herein may be understood more readily by reference to the following detailed description of specific aspects of the disclosed subject matter and the Examples included therein and to the Figures.

Before the present materials, compounds, compositions, articles, devices, and methods are disclosed and described, it is to be understood that the aspects described below are not limited to specific synthetic methods or specific reagents, as such may, of course, vary. It is also to be understood that the terminology used herein is for the purpose of describing particular aspects only and is not intended to be limiting. Also, throughout this specification, various publications are referenced. The disclosures of these publications in their entireties are hereby incorporated by reference into this application in order to more fully describe the state of the art to which the disclosed matter pertains. The references disclosed are individually and specifically incorporated by reference herein for the material contained in them that is discussed in the sentence in which the reference is relied upon.

Throughout the description and claims of this specification the word "comprise'' and other forms of the word, such as "comprising" and "comprises," means including but not limited to, and is not intended to exclude, for example, other additives, components, integers, or steps.

As used in the description and the appended claims, the singular forms "a,'' "an," and "the" include plural referents unless the context clearly dictates otherwise. Thus, for example, reference to "a composition" includes mixtures of two or more such compositions, reference to "an agent" includes mixtures of two or more such agents, reference to "the layer" includes mixtures of two or more such layers, and the like. "Optional" or "optionally" means that the subsequently described event or circumstance can or cannot occur, and that the description includes instances where the event or circumstance occurs and instances where it does not.

Certain materials, compounds, compositions, and components disclosed herein can be obtained commercially or readily synthesized using techniques generally known to those of skill in the art. For example, the starting materials and reagents used in preparing the disclosed compounds and compositions are either available from commercial suppliers or prepared by methods known to those skilled in the art.

Also, disclosed herein are materials, compounds, compositions, and components that can be used for, can be used in conjunction with, can be used in preparation for, or are products of the disclosed methods and compositions. These and other materials are disclosed herein, and it is understood that when combinations, subsets, interactions, groups, etc. of these materials are disclosed that while specific reference of each various individual and collective combinations and permutation of these compounds may not be explicitly disclosed, each is specifically contemplated and described herein. For example, if a composition is disclosed and a number of modifications that can be made to a number of components of the composition are discussed, each and every combination and permutation that are possible are specifically contemplated unless specifically indicated to the contrary. Thus, if a class of components A, B, and C are disclosed as well as a class of components D, E, and F and an example of a composition A-D is disclosed, then even if each is not individually recited, each is individually and collectively contemplated. Thus, in this example, each of the combinations A-E, A-F, B-D, B-E. B-F, C-D, C-E, and C-F are specifically contemplated and should be considered disclosed from disclosure of A, B, and C; D, E, and F; and the example combination A-D. Likewise, any subset or combination of these is also specifically contemplated and disclosed. Thus, for example, the sub-group of A-E, B-F, and C-E are specifically contemplated and should be considered disclosed from disclosure of A, B, and C; D, E, and F; and the example combination A-D. This concept applies to all aspects of this disclosure including, but not limited to, steps in methods of making and using the disclosed compositions. Thus, if there are a variety of additional steps that can be performed it is understood that each of these additional steps can be performed with any specific aspect or combination of aspects of the disclosed methods, and that each such combination is specifically contemplated and should be considered disclosed.

Reference will now be made in detail to specific aspects of the disclosed materials, compounds, compositions, articles, and methods, examples of which are illustrated in the accompanying Examples and Figures.

### I. Thermo-Responsive Blends

Described herein are thermo-responsive blends comprising at least one thermo-responsive polymer and at least one non-thermo-responsive/non-water soluble polymer. As will be described in more detail below, the properties of the thermo-responsive blends are sensitive to changes in temperature, which can be used to release cells or tissue that are immobilized on substrates comprising the blend. Each component of the blends is described below.

### a. Thermo-Responsive Polymer

The blends described herein comprise at least one thermo-responsive polymer. The term "thermo-responsive polymer" is defined herein as a polymer that has a lower critical solution temperature or an upper critical solution temperature between 0 °C and 85 °C. The critical solution temperature is defined as follows. When a certain material is mixed with water, the mixture is divided into two phases at a particular temperature because of its poor solubility, but eventually the material is completely dissolved with water to convert it to a uniform solution if it is either heated or cooled beyond a certain temperature. The certain temperature is defined as "critical solution temperature." If the uniform solution is formed when heated, the critical solution temperature is called "upper critical solution temperature. If the uniform solution is formed when cooled, it is called the "lower critical solution temperature."

In one aspect, the thermo-responsive polymers disclosed in U.S. Patent No. 5,284,766, which is incorporated by reference, can be used herein. In one aspect, the thermo-responsive polymer comprises a homopolymer or copolymer of a (meth)acrylamide, an N-substituted acrylamide, an N,N-di-substituted (meth)acrylamide, an N-substituted (meth)acrylamide, an N,N-di-substituted (meth)acrylamide, a vinyl ether, an alkyl vinyl ether, an N-vinyl caprolactam, or a combination thereof. In another aspect, the thermo-responsive polymer comprises a homopolymer or copolymer of acrylamide, methacrylamide, N-ethyl acrylamide, N-n-propyl acrylamide, N-n-propyl methacrylamide, N-isopropyl acrylamide, N-isopropyl methacrylamide, N-cyclopropyl acrylamide, N-cyclopropyl methacrylamide, N-ethoxyethyl acrylamide, N-ethoxyethyl methacrylamide, N-tetrahydrofurfuryl acrylamide, N-tetrahydrofurfuryl methacrylamide, N,N-dimethyl (meth)acrylamide, N,N-ethylmethyl acrylamide, N,N-diethyl acrylamide, 1-(1-oxo-2-propenyl)-pyrrolidine, 1-(1-oxo-2-propenyl)-piperidine, 4-(1-oxo-2-propenyl)-morpholine, 1-(1-oxo-2-methyl-2-propenyl)-pyrrolidine, 1-(1-oxo-2 -methyl-2-propenyl)-piperidine, 4-(1-oxo-2-methyl-2-propenyl)-morpholine, methyl vinyl ether, or a combination thereof. A copolymer of the above listed monomers or other monomers, a graft polymer or copolymer or a mixture of the polymers can also be employed in order to adjust the critical solution temperature, depending upon the type of cells, to enhance an interaction between the substrate and the cells/tissue, or to control the balance between the hydrophilic and hydrophobic properties of the bed material. It is also possible that the thermo-responsive polymer can be crosslinked.

In one aspect, the thermo-responsive polymer comprises a homopolymer of N-isopropyl acrylamide. In another aspect, the thermo-responsive polymer comprises polyethylene oxide, polypropylene oxide, a block copolymer of polyethylene oxide-polypropylene oxide, or a cellulose ether derivative.

In one aspect, the thermo-responsive polymer comprises at least one hydrophobic unit. The hydrophobic unit is any group or moiety that increases the hydrophobicity of the thermo-responsive polymer when compared to the hydrophobicity of the thermo-responsive polymer with no hydrophobic units. The hydrophobic unit may also increase the compatibility of the thermo-responsive polymer when it is subsequently blended with the non-thermo-responsive/non-water soluble polymer. The nature of the hydrophobic unit of the thermo-responsive polymer may be the same as the one from the non-thermo-responsive /non-water soluble The term "compatibility" is the affinity between the thermo-responsive polymer and the non-thermo-responsive/non-water soluble polymer when the two components are blended with one another. It is desirable to produce a homogeneous blend of the thermo-responsive polymer and the non-thermo-responsive/non-water soluble polymer to avoid the formation of a heterogeneous substrate. The inclusion of the hydrophobic unit in the thermo-responsive polymer increases the hydrophobicity of the thermo-responsive polymer, which in turn increases the chances of producing a homogeneous blend with the non-thermo-responsive/non-water soluble polymer that is also hydrophobic. The production of the homogeneous blend also has the benefit of being very stable and does not dissolve upon removal of cells/tissue from the substrate by extraction with a solvent such as, for example, water.

The nature and amount of hydrophobic units present in the thermo-responsive polymer can vary depending upon the selection non-thermo-responsive/non-water soluble polymer and the desired hydrophobicity of the overall blend, which can influence the release pattern of attached cells or tissue. The hydrophobic unit can be the same or different than what is used to produce the non-thermo-responsive/non-water soluble polymer. In one aspect, the hydrophobic unit is derived from H₂C=CHR, wherein R comprises an alkyl group, an aryl group, an alkoxy group, an amide group, or an ester group.

The term "alkyl group" as used herein is a branched or unbranched saturated hydrocarbon group of 1 to 24 carbon atoms, such as methyl, ethyl, n-propyl, isopropyl, n-butyl, isobutyl, t-butyl, pentyl, hexyl, heptyl, octyl, decyl, tetradecyl, hexadecyl, eicosyl, tetracosyl and the like. A "lower alkyl" group is an alkyl group containing from one to six carbon atoms. The term "aryl group" as used herein is any carbon-based aromatic group including, but not limited to, benzene, etc. The term "aryl" also includes "heteroaryl group," which is defined as an group that has at least ane heteroatom incorporated within the ring of the aromatic group, Examples of heteroatoms include, but are not limited to, nitrogen, oxygen, sulfur, and phosphorus. The aryl group can be substituted or unsubstituted. The aryl group can be substituted with one or more groups including, but not limited to, alkyl, alkynyl, alkenyl, aryl, halide, nitro, amino, ester, ketone, aldehyde, hydroxy, carboxylic acid, or alkoxy. The terms alkoxy, ester, and amide are represented by the formulae -OR, -C(O)OR, and -C(O)NRR', respectively, where R and R' can be an alkyl group or aryl group as defined above. In one aspect, the hydrophobic unit is derived from styrene.

The thermo-responsive polymer can be a copolymer, graft polymer, or block copolymer comprising the hydrophobic unit. In one aspect, the thermo-responsive polymer comprises a copolymer of (1) N-isopropyl acrylamide, polyethylene oxide, a polypropylene oxide, or a block copolymer of polyethylene oxide-polypropylene oxide, or a combination thereof and (2) styrene. In another aspect, the thermo-responsive polymer comprises a block copolymer of poly(propylene oxide)-poly(ethylene oxide)-polystyrene, a block copolymer of poly(propylene oxide)-polystyrene, or a block copolymer of poly(N-isopropylacrylamide)-polystyrene.

The amount of hydrophobic unit present in the thermo-responsive polymer can vary. When the thermo-responsive polymer contains a hydrophobic unit, the thermo-responsive polymer can be prepared by techniques known in the art including, but not limited to, atom transfer radical polymerization (ATRP), reversible addition-fragmentation transfer polymerization (RAFT), nitroxy-mediated polymerization (NMP), anionic polymerization, or by reactive extrusion.

### b. Non-Thermo-Responsive/Non-water soluble polymer

The blends described herein also contain a non-thermo-responsive/non-water soluble polymer. Non-thermo-responsive/non-water soluble polymers are polymers that are not thermo-responsive as defined above and have nominal to no solubility in water.

As will be discussed below, the non-thermo-responsive/non-water soluble polymer promotes cell adhesion. This is due to the fact that the non-thermo-responsive/non-water soluble is hydrophobic, and cells generally adhere to hydrophobic surfaces. In one aspect, the non-thermo-responsive/non-water soluble polymer comprises a polyester, a polyamide, a polycarbonate, a polycaprolactone, a polyurethane, a polyimide, a polyamide-imide, a poly-lactide-co-glycolide, a polyolefin, a cyclic polyolefin, a polysulfone, a poly (meth)acrylate polymer, a poly vinyl polymer, or a combination thereof. In one aspect, the non -thermo-responsive/non-water soluble polymer is transparent.

### c. Preparation of Blends

The blends described herein can be prepared by techniques known in the art. In one aspect, the blends can be prepared by compounding processes at high temperature. The thermo-responsive and non-thermo-responsive/non-water soluble polymers can be mixed at high temperature so that blends can be obtained by extrusion. For example poly-N-isopropyl acrylamide (the thermo-responsive polymer) is stable up to 270 °C, which compares favorably with the thermal stability of polystyrene (280°C) (the non-thermo-responsive/non -water soluble polymer). As will be discussed below, the blends can be molded to form shaped products with current injection molding machines. The amount of thermo-responsive polymer and non-thermo-responsive/non-water soluble polymer used to prepare the blend will vary depending upon the polymers selected and the desired release pattern of the cells. By varying the amount of each polymer, it is possible to increase or decrease cell adhesion as well as fine-tune cell release upon changing the temperature. In one aspect, blend comprises from 1% to 99% by weight thermo-responsive polymer, from 1 to 50% by weight thermo-responsive polymer, or from 1 to 25% by weight thermo-responsive polymer.

### II. Substrates for Cell/Tissue Immobilization

Described herein are substrates for immobilizing cells or tissues using the blends described herein. Upon immobilization of the cells or tissue, numerous applications are contemplated. These applications will be described below.

In one aspect, described herein is substrate for immobilizing cells or tissue comprising:
a network of nanofibers, wherein the network of nanofibers comprises one or more thermo-responsive blends described herein; and
a base substrate comprising a first outer surface, wherein the network of nanofibers is adjacent to the first outer surface of the base substrate.

In another aspect, described herein is a substrate for immobilizing cells or tissue comprising:
a network of nanofibers; and
a base substrate comprising a first outer surface, wherein the network of nanofibers is adjacent to the first outer surface of the base substrate, and wherein the base substrate comprises one or more thermo-responsive blends described herein.

Each component of the substrates is described below.

### a. Network of Nanofibers

The term "nanofiber" as used herein means a polymer fine fiber comprising a diameter of about 1000 nanometers or less. The term "network'' as used herein means a random or oriented distribution of nanofibers in space that is controlled to form an interconnecting net with spacing between fibers selected to promote growth and culture stability. The network has small spaces between the fibers comprising the network forming pores or channels in the network. The size of the pores or channels can vary depending upon the cell or tissue to be immobilized. In one aspect, the pore size of the nanofiber network is greater than 0.2 microns. In another aspect, the pore size is less than 1 micron. In a further aspect, the pore size is from 0.2 microns to 300 microns. The network can comprise a single layer of nanofibers, a single layer formed by a continuous nanofiber, multiple layers of nanofibers, multiple layers formed by a continuous nanofiber, or mat. The network may be unwoven or net. Physical properties of the network include, but are not limited to, texture, rugosity, adhesivity, porosity, solidity, elasticity, geometry, intereconnectivity, surface to volume ratio, fiber diameter, fiber solubility/insolubility, hydrophilicity/hydrophobicity, fibril density, and fiber orientation.

In certain aspects, the network of nanofibers comprises at least one thermo-responsive blend described herein. It is also possible to have layers of two or more different nanofiber networks. The different layers can be composed of different thermo-responsive blends described herein or, in the alternative, one or more of the layers can be composed of polymers other than the blends described herein. For example, nanofibers can be derived from natural or synthetic fibers. Examples of natural fibers include, but are not limited to, a protein, a polysaccharide, a cellulose derivative, or a mixture thereof. Examples of synthetic fibers include, but are not limited to, a polyester, a polyamide, or a mixture thereof.

In other aspects, polymer materials that can be used to produce nanofiber networks that do not contain the blends described herein include both polymer and condensation polymer materials such as polyolefin, cyclic polyolefin, polyacetal, polyamide, polyester, polycarbonate, cellulose ether and ester, polyalkylene sulfide, polyarylene oxide, polyalkylene oxide, copolymers and block copolymers of alkylene oxide, polyvinylcarbazole, polysulfone, modified polysulfone polymers and mixtures thereof. Preferred materials that fall within these generic classes include polyethylene, poly(epsilon-caprolactone), a polylactide, a polyglycolide, a polylactide-co-glycolide, polypropylene, polysiloxane, poly(vinylchloride), polyvinylpyrrolidone, polyvinyl acetate, polymethylmethacrylate (and other (meth)acrylic resins), poly (meth)acrylamide, polystyrene, and copolymers thereof (including ABA type block copolymers), poly(vinylidene fluoride), poly(vinylidene chloride), polyvinyl alcohol in various degrees of hydrolysis (87% to 99.5%) in crosslinked and non-crosslinked forms.

In certain aspects, when the base substrate comprises one or more thermo-responsive blends described herein, the nanofiber network may or may not contain a thermo-responsive blend described herein In the case when the nanofiber network does not contain a thermo-responsive blend described herein, the nanofiber can be composed of any of the polymers described above.

The nanofibers can be fabricated using techniques known in the art. Polymer selection and/or the process by which the nanofibers are fabricated and/or directed and oriented onto a substrate allow for specific selection and manipulation of physical properties of the nanofiber network. Physical properties of the nanofiber network, including fiber size, fiber diameter, fiber spacing, matrix density, fiber texture and elasticity, can be important considerations for organizing the cytoskeletal networks in cells and the exposure of cell signaling motifs in extracellular matrix proteins. Physical properties of the nanofiber network that can be engineered to desired parameters include, but are not limited to, texture, rugosity, adhesivity, porosity, solidity, elasticity, geometry, interconnectivity, surface to volume ratio, fiber size, fiber diameter, fiber solubility/insolubility, hydrophilicity/hydrophobicity, and fibril density.

One or more of the physical properties of the nanofiber network can be varied and/or modified to create a specifically defined environment for cell/tissue immobilization. For example, porosity of the nanofiber network can be engineered to enhance diffusion of ions, metabolites, and/or bioactive molecules and/or allow cells to penetrate and permeate the nanofiber network to grow in an environment that promotes multipoint attachments between the cells and the nanofiber network. Interconnectivity of the nanofiber network can be engineered to facilitate cell-cell contacts. Elasticity of the nanofiber network can be increased or decreased by adding a bioactive molecule to the polymer solution from which the nanofibers are fabricated. It is also possible to produce nanofibers that are hollow or have a core with a sheath.

Texture and rugosity of the nanofiber network can be engineered to promote attachment of cells. For example, homogeneous or heterogeneous nanofibers can be selected to optimize growth or differentiation activity of the cells. In one aspect, the nanofiber network comprises multiple nanofibers having different diameters and/or multiple nanofibers fabricated from different polymers. In other aspects, the solubility or insolubility of the nanofibers of the nanofiber network can be engineered to control the release of bioactive molecules that can be incorporated into the nanofiber network. For example, the rate of release of bioactive molecules is determined by the rate of biodegradation or biodissolution of the nanofibers of the nanofiber network. In other aspects, the hydrophobicity and hydrophilicity of the nanofiber network can be engineered to promote specific cell spacing.

In one aspect, the nanofiber network can be produced by charging techniques such as, for example, corona charging and tribocharging. In another aspect, the nanofibers can be prepared by electrospinning techniques. The electrospinning process uses an electric field to control the formation and deposition of polymers. A polymer solution is injected with an electrical potential. The electrical potential creates a charge imbalance that leads to the ejection of a polymer solution stream from the tip of an emitter such as a needle. The polymer jet within the electric field is directed toward a grounded substrate, during which time the solvent evaporates and fibers are formed. The resulting single continuous filament collects as a nonwoven fabric on the base substrate.

The nanofiber networks can be produced having random or directed orientations. Random fibers can be assembled into layered surfaces. In one aspect, the nanofibers comprise a random distribution of fine fibers that can be bonded to form an interlocking network. The nanofiber interlocking networks have relatively small spaces between the fibers. Such spaces form pores or channels in the nanofiber network allowing for diffusion of ions, metabolites, proteins, and/or bioactive molecules as well as cells to penetrate and permeate the network and grow in an environment that promotes multipoint attachments between cells and the nanofibers.

In other aspects, nanofiber networks can be electrospun in an oriented manner. Such oriented electrospinning allows for the fabrication of a nanofiber network comprising a single layer of nanofibers or a single layer formed by a continuous nanofiber, wherein the network has a height of the diameter of a single nanofiber. Physical properties including porosity, solidity, fibril density, texture, rugosity, and fiber orientation of the single layer network can be selected by controlling the direction and/or orientation of the nanofiber onto the substrate during the electrospinning process. Preferably the pore size allows cells to penetrate and/or migrate through a single layer or multi-layer nanofiber network.

The layering of individual single layer nanofiber networks can form channels, which allow diffusion of ions, metabolites, proteins, and/or bioactive molecules as well as permit cells to penetrate the nanofiber network and grow in an environment that promotes multipoint attachments between the cells and the nanofiber network.

The morphology and physical properties of the nanofiber network can vary depending upon, among other things, the selection of the polymer, the conformation of the polymer chain, and the solvent used. In one aspect, phase separation techniques can be used to fabricate the nanofiber network. The phase separation process generally involves polymer dissolution, phase separation and gelation, solvent extraction from the gel with water, freezing, and then freeze drying under a vacuum. By varying the ratio of polymers and the solvents, it is possible to control the topography of the nanofibers.

### b. Base Substrate

The term "base substrate" as used herein means any surface on which the network of nanofibers can be deposited. The base substrate can be any surface that offers structural support for the deposited network of nanofibers. In one aspect, the base substrate can comprise glass, cellulose, or plastic. In another aspect, the base substrate can be a film, a woven mat, a non-woven mat, or an article.

The base substrate can be porous or non-porous. Porosity of the base substrate is determined by cellular penetration. A cell is able to penetrate a porous substrate but is not able to penetrate a non-porous substrate. Depending upon the porosity of the nanofiber network, the base substrate can have pores that are greater or smaller in diameter to the pores present in the nanofiber network. It is contemplated that cells can penetrate and be retained by the base substrate and/or the network of nanofibers. The size of the pores in the base substrate can vary depending upon the cell or tissue to be immobilized. In one aspect, the pore size is greater than 0.2 microns. In another aspect, the pore size is less than 1 micron. In a further aspect, the pore size is from 0.2 microns to 300 microns.

In one aspect, the base substrate is composed of one or more blends described herein. In another aspect, the base substrate is composed of one or more polymers that does not include the thermo-responsive blend. Any of the polymers described above for producing nanofibers can be used to produce the base substrate. Examples of such polymers include, but are not limited to, a polyolefin, cyclic polyolefin, polyacetal, polyamide, polyester, polycarbonate, cellulose ether and ester, polyalkylene sulfide, polyarylene oxide, polyalkylene oxide, copolymers and block copolymers of alkylene oxide, polyvinylcarbazole, polysulfone, modified polysulfone polymers and mixtures thereof. Preferred materials that fall within these generic classes include polyethylene, poly(epsilon-caprolactone), a polylactide, a polyglycolide, a polylactide-co-glycolide, polypropylene, polysiloxane, poly(vinylchloride), polyvinylpyrrolidone, polyvinyl acetate, polymethylmethacrylate (and other (meth)acrylic resins), poly (meth)acrylamide, polystyrene, and copolymers thereof (including ABA type block copolymers), poly(vinylidene fluoride), poly(vinylidene chloride), polyvinyl alcohol in various degrees of hydrolysis (87% to 99.5%) in crosslinked and non-crosslinked forms. It is contemplated that the base substrate can be composed of layers of different polymers or composed of a blend of two or more polymers. Any of the polymers described above can be woven or non-woven to produce the base substrate. For example, the base substrate can be composed of Nylon fibers woven into a mat.

It is also contemplated that the base substrate can be used alone in the absence of a nanofiber network to immobilize cells. For example, the substrate can comprise at least one blend described herein. In another aspect, the substrate can be composed solely of one or more blends described herein. The base substrate can be manufactured into any desirable shape or article using techniques known in the art, which is described in more detail below.

### c. Bioactive Molecules

The nanofiber network and/or the base substrate can comprise one or more bioactive molecules. In one aspect, the network of nanofibers or base substrate comprises one or more compounds for enhancing cell/tissue growth. In another aspect, the nanofiber or base substrate further comprises a compound that promotes attachment of a cell or tissue to the nanofiber or substrate.

Bioactive molecules include human or veterinary therapeutics, nutraceuticals, vitamins, salts, electrolytes, amino acids, peptides, polypeptides, proteins, carbohydrates, lipids, polysaccharides, nucleic acids, nucleotides, polynucelotides, glycoproteins, lipoproteins, glycolipids, glycosaminoglycans, proteoglycans, growth factors, differentiation factors, hormones, neurotransmitters, pheromones, chalones, prostaglandins, immunoglobulins, monokines and other cytokines, humectants, minerals, electrically and magnetically reactive materials, light sensitive materials, antioxidants, molecules that may be metabolized as a source of cellular energy, antigens, and any molecules that can cause a cellular or physiological response. Any combination of molecules can be used, as well as agonists or antagonists of these molecules. Glycoaminoglycans include glycoproteins, proteoglycans, and hyaluronan. Polysaccharides include cellulose, starch, alginic acid, chytosan, or hyaluronan. Cytokines include, but are not limited to, cardiotrophin, stromal cell derived factor, macrophage derived chemokine (MDC), melanoma growth stimulatory activity (MGSA), macrophage inflammatory proteins 1 alpha (MIP-1 alpha), 2, 3 alpha, 3 beta, 4 and 5, interleukin (IL) 1, IL-2, IL-3, IL-4, IL-5, IL-6, IL-7, IL-8, IL-9, IL-10, IL- 11, IL-12, IL-13, TNF-alpha, and TNF-beta. Immunoglobulins useful in the present invention include, but are not limited to, IgG, IgA, IgM, IgD, IgE, and mixtures thereof. Amino acids, peptides, polypeptides, and proteins can include any type of such molecules of any size and complexity as well as combinations of such molecules. Examples include, but are not limited to, structural proteins, enzymes, and peptide hormones.

The term bioactive molecule also includes fibrous proteins, adhesion proteins, adhesive compounds, deadhesive compounds, and targeting compounds. Fibrous proteins include collagen and elastin. Adhesion/deadhesion compounds include fibronectin, laminin, thrombospondin and tenascin C. Adhesive proteins actin, fibrin, fibrinogen, fibronectin, vitronectin, laminin, cadherins, selectins, intracellular adhesion molecules 1, 2, and 3, and cell-matrix adhesion receptors including but not limited to integrins such as α₅β₁, α₆β₁, α₇β₁, α₄β₂, α₂β₃, and α₆β₄.

The term bioactive molecule also includes leptin, leukemia inhibitory factor (LIF), RGD peptide, tumor necrosis factor alpha and beta, endostatin, angiostatin, thrombospondin, osteogenic protein-1, bone morphogenic proteins 2 and 7, osteonectin, somatomedin-like peptide, osteocalcin, interferon alpha, interferon alpha A, interferon beta, interferon gamma, interferon 1 alpha, and interleukins 2, 3, 4, 5 6, 7, 8, 9, 10, 11, 12, 13, 15, 16, 17 and 18.

The term "growth factor" as used herein means a bioactive molecule that promotes the proliferation of a cell or tissue. Growth factors useful in the present invention include, but are not limited to, transforming growth factor-alpha. (TGF-alpha), transforming growth factor-beta. (TGF-beta), platelet-derived growth factors including the AA, AB and BB isoforms (PDGF), fibroblast growth factors (FGF), including FGF acidic isoforms 1 and 2, FGF basic form 2, and FGF 4, 8, 9 and 10, nerve growth factors (NGF) including NGF 2.5s, NGF 7.0s and beta NGF and neurotrophins, brain derived neurotrophic factor, cartilage derived factor, bone growth factors (BGF), basic fibroblast growth factor, insulin-like growth factor (IGF), vascular endothelial growth factor (VEGF), EG-VEGF, VEGF-related protein, Bv8, VEGF-E, granulocyte colony stimulating factor (G-CSF), insulin like growth factor (IGF) I and II, hepatocyte growth factor (HGF), glial neurotrophic growth factor (GDNF), stem cell factor (SCF), keratinocyte growth factor (KGF), transforming growth factors (TGF), including TGFs alpha, beta, beta1, beta2, and beta3, skeletal growth factor, bone matrix derived growth factors, and bone derived growth factors and mixtures thereof. Some growth factors can also promote differentiation of a cell or tissue. TGF, for example, can promote growth and/or differentiation of a cell or tissue. Some preferred growth factors include VEGF, NGFs, PDGF-AA, PDGF-BB, PDGF-AB, FGFb, FGFa, HGF, and BGF.

The term "differentiation factor" as used herein means a bioactive molecule that promotes the differentiation of cells or tissues. The term includes, but is not limited to, neurotrophin, colony stimulating factor (CSF), or transforming growth factor. CSF includes granulocyte-CSF, macrophage-CSF, granulocyte-macrophage-CSF, erythropoietin, and IL-3. Some differentiation factors may also promote the growth of a cell or tissue. TGF and IL-3, for example, can promote differentiation and/or growth of cells.

The term "adhesive compound" as used herein means a bioactive molecule that promotes attachment of a cell or tissue to a fiber surface comprising the adhesive compound. Examples of adhesive compounds include, but are not limited to, fibronectin, vitronectin, and laminin.

The term "deadhesive compound" as used herein means a bioactive molecule that promotes the detachment of a cell or tissue from a fiber comprising the deadhesive compound. Examples of deadhesive compounds include, but are not limited to, thrombospondin and tenascin C.

The term "targeting compound" as used herein means a bioactive molecule that functions as a signaling molecule inducing recruitment and/or attachment of cells or tissues to a fiber comprising the targeting compound. Examples of targeting compounds and their cognate receptors include attachment peptides including RGD peptide derived from fibronectin and integrins, growth factors including EGF and EGF receptor, and hormones including insulin and insulin receptor.

The bioactive molecules can be incorporated into the nanofiber network or the base substrate during fabrication of the network or substrate or can be attached to a surface of the network or substrate via a functional group. In certain aspects, one or more functional groups can be incorporated on the outside surface of the nanofibers or base substrate. These functionalized surfaces can bind a peptide, polypeptide, lipid, carbohydrate, polysaccharide, amino acid, nucleotide, nucleic acid, polynucleotide, or other bioactive molecules to the surface of the nanofiber or base substrate. In one aspect, the functional groups are deposited on the outside surface of the nanofiber or base substrate by plasma deposition. Plasma deposition creates local plasmas at the surface of the nanofiber or base substrate. The treated surface is then reacted with gaseous molecules, such as for example, allylamine and/or allyl alcohol, in a reaction chamber. In another aspect, the functional groups are introduced onto the surface of the nanofibers during the electrospinning process. For example, dodecyl amine, dodecyl aldehyde, dodecyl thiol, or dodecyl alcohol can be added to the polymer solution. The polymer solution is then electrospun into nanofibers in which a portion of the added amines, aldehydes, sulphydryl, or alcohol moieties, respectively, are exposed on the outside surface of the nanofibers.

### III. Preparation of Substrates for Cell/Tissue Immobilization

The nanofiber network can be deposited on the base substrate using techniques known in the art. In one aspect, the nanofiber network can be produced and deposited on the base substrate by charging techniques such as, for example, corona charging and tribocharging. Alternatively, the nanofiber network can be electrospun onto the base substrate such that the nanofiber network is adjacent to the base substrate. In other aspects, a preformed nanofiber network can be attached to the base substrate with the use of an adhesive.

The term "adjacent'' as used herein includes the intimate contact between the nanofiber network and the surface of the base substrate. The term "adjacent" also includes one or more layers interposed between the nanofiber network and the base substrate. For example, an adhesion protein can be deposited on the outer surface of the base substrate prior to depositing the nanofiber network on the base substrate. In one aspect, cells or tissue are not interposed between the nanofiber network and the base substrate. As described above, electrospinning can be used to produce nanofibers with different properties and orientations as desired. In general, although not prohibited, the other exposed surface of the base substrate does not have any components adjacent to the other exposed surface. Upon deposition of the nanofibers on the base substrate, the nanofibers are evenly distributed on the base substrate at a uniform thickness.

It is also contemplated that two or more nanofiber networks can be layered on the base substrate. For example, different nano- and/or micro-environments that promote cellular activity of a particular cell or tissue can be constructed by layering different nanofiber networks that have selected physical and/or chemical properties. The physical and/or chemical properties can be engineered into the individual nanofiber networks as described above. The layering of individual nanofiber networks can form channels that allow diffusion of ions, metabolites, proteins, and/or bioactive molecules as well as permit cells to penetrate the substrate and grow in an environment that promotes multipoint attachments between the cells and the nanofiber network.

### IV. Articles

Any of the blends described herein can be molded into various shapes and sizes depending upon the desired end-use of the article. For example, the blend can be extruded using techniques known in the art. In one aspect, the blend can be used to make a single well culture plate, a multiwell culture plate, a microplate, a chambered culture slide, a multi-chambered culture slide, a coverslip, a cup, a flask, a tube, a bottle, a perfusion chamber, a bioreactor, or a fermentor. It is contemplated that the article can be the base substrate that can receive a nanofiber network. For example, the blend can be extruded to produce a culture plate, at which point one or more nanofiber networks can be layered on the culture plate.

Articles produced by the blends described herein possess thermo-responsive properties throughout the entire article. Thus, the thermo-responsive properties will be maintained even if the article is scratched or damaged at its surface. In general, coated products for cell culturing are designed for a single use. If the coating is damaged during handling or cell culturing, the thermo-responsive property may be lost. In the articles described herein, the bulk of the article is modified to induce the thermo-responsive property at the surface; therefore, scratches are not detrimental to the function of the article. Therefore, permanent, durable and reversible thermo-responsive properties are expected with the articles produced by the blends described herein.

### V. Kits

In another aspect, described herein is a kit comprising a network of nanofibers and a base substrate. Any of the nanofiber networks and base substrates described above can be used herein. In one aspect, one or more pre-manufactured nanofiber networks can be individually wrapped and sterilized. After removal from the packaging, one or more nanofiber networks can be assembled manually or mechanically on the base substrate. In the case of multiple nanofiber networks, each nanofiber network can be applied to the base substrate layer by layer to form a multilayered assembly. The base substrate can be an article such that it is shaped to receive the nanofiber network.

### VI. Applications

The substrates described herein are used to immobilize cells or tissues. The term "immobilization" as used herein is the ability of the substrate to retain the cell or tissue. Immobilization can range from completely retaining the cell or tissue such that the cell or tissue is locked in position within the nanofiber network or base substrate to a situation where the cell or tissue can freely permeate the nanofiber network or base substrate. The incorporation of bioactive molecules into the nanofiber network or base substrate can determine the degree of immobilization of the cell or tissue on the substrate.

The substrates described herein can be used in a number of applications, which are described below. It is contemplated that the substrates can be used in many known applications employing nanofibers including, but not limited to, filter applications, pharmaceutical applications, cell culture, tissue culture, and tissue engineering. It is contemplated one or more cell types can be deposited on the substrate. The cells can be deposited on the substrate using techniques known in the art.

In one aspect, described herein is a method for growing a plurality of cells, comprising (a) depositing a parent set of cells on a substrate described herein, and (b) culturing the substrate with the deposited cells to promote the growth of the cells.

In another aspect, described herein is a method for differentiating cells, comprising (a) depositing a parent set of cells on a substrate described herein, and (b) culturing the assembly to promote differentiation of the cells.

Many types of cells can be immobilized on the substrate including, but not limited to, stem cells, committed stem cells, differentiated cells, and tumor cells. Examples of stem cells include, but are not limited to, embryonic stem cells, bone marrow stem cells and umbilical cord stem cells. Other examples of cells used in various embodiments include, but are not limited to, osteoblasts, myoblasts, neuroblasts, fibroblasts, glioblasts, germ cells, hepatocytes, chondrocytes, keratinocytes, smooth muscle cells, cardiac muscle cells, connective tissue cells, glial cells, epithelial cells, endothelial cells, hormone-secreting cells, cells of the immune system, and neurons.

Cells useful herein can be cultured *in vitro,* derived from a natural source, genetically engineered, or produced by any other means. Any source of prokaryotic or eukaryotic cells can be used.

Atypical or abnormal cells such as tumor cells can also be used herein. Tumor cells cultured on substrates described herein can provide more accurate representations of the native tumor environment in the body for the assessment of drug treatments. Growth of tumor cells on the substrates described herein can facilitate characterization of biochemical pathways and activities of the tumor, including gene expression, receptor expression, and polypeptide production, in an in vivo-like environment allowing for the development of drugs that specifically target the tumor.

Cells that have been genetically engineered can also be used herein. The engineering involves programming the cell to express one or more genes, repressing the expression of one or more genes, or both. Genetic engineering can involve, for example, adding or removing genetic material to or from a cell, altering existing genetic material, or both. Embodiments in which cells are transfected or otherwise engineered to express a gene can use transiently or permanently transfected genes, or both. Gene sequences may be full or partial length, cloned or naturally occurring.

By varying and/or modifying selected physical and/or chemical properties of the substrate, the substrate can be engineered to promote cellular growth of a particular cell or tissue. The physical properties and/or characteristics of the substrate including, but not limited to, texture, rugosity, adhesivity, porosity, elasticity, solidity, geometry, and fibril density can be varied and/or modified to promote a desired cellular activity, including growth and/or differentiation. Specific nano- and/or micro-environments can be engineered within the substrate. For example, the porosity and fibril density of the substrate can be varied and/or modified to allow a cell to penetrate the substrate and grow in a three dimensional environment. Any of the bioactive molecules described herein can be engineered into the substrate either isotropically or as gradients to promote desired cellular activity, including cell adhesion, growth, and/or differentiation. The physical and/or chemical properties of the substrate, including growth and differentiation factors, on which such cells are grown can be engineered to mimic the native *in vivo* nano- or micro-environments.

With designed patterns, the spatial organization of the cells in two and three dimensions can be obtained. By creating specific patterns of surface chemistry, cell behavior can be confined within physical or chemical ultrastructures, which can be used to control cellular activity such as cell growth and/or proliferation.

In certain instances, it is desirable to remove the cells or tissue from the For example, it would be desirable to harvest stem cells that have been growing on the substrates described herein at 37 °C. In general, the harvesting of cells immobilized on substrates involves invasive techniques such as the use of enzymes or chemicals. The use of these techniques can destroy valuable cells or, in the alternative, introduce impurities that can contaminate the cells. The blends described herein permit the facile release of cells and tissues immobilized on the substrate. In order to detach the immobilized cells, the substrate is either heated or cooled to exceed the upper or lower critical solution temperature. After the heating or cooling step, the substrate can be rinsed with a solution (e.g., an isotonic solution) to collect the cells.

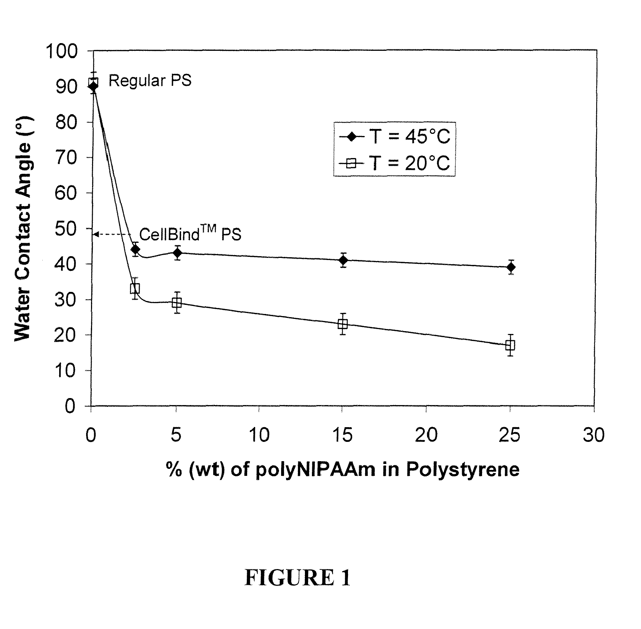

The temperature at which the cells can be released from the substrate can vary depending upon the selection of the thermo-responsive polymer, the non-thermo-responsive/non-water soluble polymer and the relative amounts of each polymer present in the blend. Not wishing to be bound by theory, hydrophobic surfaces promote cell adhesion and hydrophilic surfaces prevent cell adhesion. The degree of hydrophobicity can be measured by water contact angle, where an increase in water contact angle indicates an increase in hydrophobicity. The presence of the thermo-responsive polymer reduces the hydrophobicity (i.e., water contact angle) of the blend as a function of temperature. For example, the water contact angle of a blend composed of poly-N-isopropylacrylamide (of 2.5 wt %) and polystyrene decreases from 44° to 33° when the temperature is reduced from 45 to 20 °C (Figure 1). In another example, the water contact angle of a blend composed of poly-N-isopropylacrylamide (25 wt %) and polystyrene decreases from 39° to 17° when the temperature is reduced from 45 to 20 °C (Figure 1). In these examples, varying the amount of thermo-responsive polymer can alter the release pattern of the cells or tissue from the substrate as a function of temperature. Thus, release profiles can be established over a range of different temperatures by selecting different thermo-responsive polymers and hydrophobic polymers at varying amounts. This is particularly attractive, as some cells may be sensitive to certain temperatures.

In another aspect, described herein is method for growing tissue, comprising (a) depositing a parent set of cells that are a precursor to the tissue on a substrate described herein, and (b) culturing the substrate with the deposited cells to promote the growth of the tissue. It is also contemplated that viable cells can be deposited on the substrates described herein and cultured under conditions that promote tissue growth. Tissue grown (i.e., engineering) from any of the cells described above is contemplated with the substrates described herein. The supports described herein can support many different kinds of precursor cells, and the substrates can guide the development of new tissue. The production of tissues has numerous applications in wound healing. Depending upon the selection of materials used to produce the nanofibers and base substrate, tissue growth can be performed *in vivo* or *ex vivo.*

In certain instances, it is desirable to remove the cells or tissue from the substrate. For example, it would be desirable to harvest stem cells that have been growing on the substrates described herein. Invasive techniques known in the art for removing cells include, but are not limited to, mechanical scraping, sonication, chemical/enzymatic treatment, or a combination thereof. Other techniques involve adjusting the pH or temperature or the addition of ions to release attached cells.

In another aspect, described herein are methods for determining an interaction between a known cell line and a drug, comprising (a) depositing the known cell line on a substrate described herein; (b) contacting the deposited cells with the drug; and (c) identifying a response produced by the deposited cells upon contact with the drug.

With a known cell line immobilized on the substrates described herein, it is possible to screen the activity of several drugs when the drug interacts with the immobilized cells. Depending upon the cells and drugs to be tested, the cell-drug interaction can be detected and measured using a variety of techniques. For example, the cell may metabolize the drug to produce metabolites that can be readily detected. Alternatively, the drug can induce the cells to produce proteins or other biomolecules. The substrates described herein provide an environment for the cells to more closely mimic the *in vivo* nature of the cells in an *ex vivo* environment. The substrates can be used in high throughput applications for analyzing drug/cell interactions. High throughput applications utilize multiwell tissue culture chambers with densities up to about 1536 wells per plate. Thus, increasing the population of cells per well would serve to increase the measured signals.

In another aspect, described herein are methods for separating a compound present in a solution, comprising (a) contacting the solution with a substrate described herein, wherein the compound is immobilized on the substrate; and (b) removing the immobilized compound from the substrate. The nanofiber network and/or base substrate can be modified to immobilize particular biomolecules in solution. In general, a solution composed of one or more biomolecules is contacted with the substrate, at which time the biomolecule is immobilized on the substrate. The bound biomolecule can then be released from the substrate with a solvent. The substrate can be modified so that the substrate forms a covalent or non-covalent (e.g., ionic, electrostatic dipole-dipole, Van Der Waals interactions) bond with the biomolecule. In another aspect, cells can be purified. For example, by measuring the electric properties of a single individual cell immobilized on the substrate, it is possible to sort/purify a population of cells by their different intrinsic electric properties. This application can be of particular interest in stem cells, where it is desirable to harvest large quantities of pure stem cells.

### EXAMPLES

The following examples are set forth below to illustrate the methods and results according to the disclosed subject matter. These examples are not intended to be inclusive of all aspects of the subject matter disclosed herein, but rather to illustrate representative methods and results. These examples are not intended to exclude equivalents and variations of the present invention which are apparent to one skilled in the art.

Efforts have been made to ensure accuracy with respect to numbers (e.g., amounts, temperature, etc.) but some errors and deviations should be accounted for. Unless indicated otherwise, parts are parts by weight, temperature is in °C or is at ambient temperature, and pressure is at or near atmospheric. There are numerous variations and combinations of reaction conditions, e.g., component concentrations, temperatures, pressures and other reaction ranges and conditions that can be used to optimize the product purity and yield obtained from the described process. Only reasonable and routine experimentation will be required to optimize such process conditions.

### EXAMPLE 1-Preparation of Blends of Polystyrene and PolyNIPAAm

Blends of polystyrene and poly(N-isopropylacrylamide (polyNIPAAm; Mₙ = 20,000-25,000, ref Aldrich 535311) were prepared by mixing the polymers in solution and evaporating the solvent to obtain solid blends. Other mixing techniques such as extrusion can be used as the thermal properties (e.g., thermal stability) of the two polymers are very similar. In such conditions, the current forming techniques of polystyrene such as injection molding will be not significantly affected by the presence of the polyNIPAAm additive.

The variation of the water contact angle on blends composed of polystyrene and polyNipAAm is illustrated in Figure 1. The water contact angle was measured at two different temperatures for different compositions of blends, the amount of polyNIPAAm varying from 0 to 25 wt%. The polystyrene/polyNIP AAm blends were significantly more hydrophobic at 45°C than at 20 °C. When the blend contains 2.5 wt% of polyNIPAAm, the water contact angle was reduced from 44° to 33° when the temperature was reduced from 45 to 20 °C. At 25 wt% of polyNIPAAm, the contact angle was reduced from 39° to 17° for the same temperature variation.

Figure 1 shows that a thermo-responsive effect was obtained approximately at 2.5 wt% of polyNIPAAm. In the "hydrophobic" state characterized at 45 °C, the wettability of the blends was very close to the wettability of the CellBind^{™} product (Coming), which indicates that the blend has the appropriate properties for cell culturing. Conversely, regular polystyrene (PS) was too hydrophobic (water contact angle of 90°) and had no thermo-responsive properties.

### EXAMPLE 2-Preparation and Characterization of Blends Composed of Copolymers

The thermo-responsive properties of the surface of polymer blends were investigated using the following thermo-responsive segments: (poly(N-isopropylacrylamide) (PNIPAAM-LCST 32 °C), polypropylene oxide (PPO-LCST 10-15°C) Aldrich reference no. 202355, Mn 4,000 g/mol, and F Pluronic 127^{®} (Sigma reference no. P2443), poly(ethylene oxide)-block-poly(propylene oxide)-block-poly(ethylene oxide) (P122-LCST 27°C), Mn 12,600g/mol. A polystyrene (PS)-block-PNIPAAM copolymer (PSPNIPAAM2) was prepared by successive atom transfer radical polymerization (ATRP) of styrene at 80 °C, using dimethyl dibromoheptanedioate as initiator and copper bromide/ pentamethyldiethylenetriamine as catalyst, and N-isopropylacrylamide at 25 °C using copper chloride/ tris[2-(dimethylamino)ethyl]amine as catalyst using the techniques described in U.S. Patent No. 5,763,548, which is incorporated by reference. P127-bock-PS (P127PS1) and PPO-block-PS were by functionalization of commercially available respectively hydroxy-terminated PPO and hydroxy-terminated Pluronic 127^{®} with bromoisobutyrate groups using bromoisobutyryl bromide and triethylamine in anisole, followed by the ATRP of styrene at 80 °C using copper bromide/pentamethyldiethylenetriamine as catalyst. The block copolymers exhibited low polydispersity indices (PDI), which were below 1.4 (Table I).

**Table 1**

| Sample | Mn (g/mol) | Polydispersity index (PDI) | Molar composition | Thermo-responsive Polymer content (wt%) |
|---|---|---|---|---|
| PPOPS1 | 18100 | 1.3 | (St)₅₄-(PO)₆₉-(St)₅₄ | 45 |
| PPOPS2 | 11800 | 1.4 | (St)₂₃-(PO)₆₉-(St)₂₃ | 26 |
| P127PS1 | 41300 | 1.32 | (St)₁₄₆-(EO)₁₀₁-(PO)₅₆-(EO)₁₀₁-(St)₁₄₆ | 29 |
| PSPNIPAAM2 | Not measured | Not measured | (NIPAAM)₁₉₄-(St)₂₁₃-(NIPAAM)₁₉₄ | 66 |

| | | | | |
|---|---|---|---|---|
| St : styrene repetitive units PO : propylene oxide repetitive units EO : ethylene oxide repetitive units NIPAAM : N-isopropylacrylamide repetitive units | | | | |

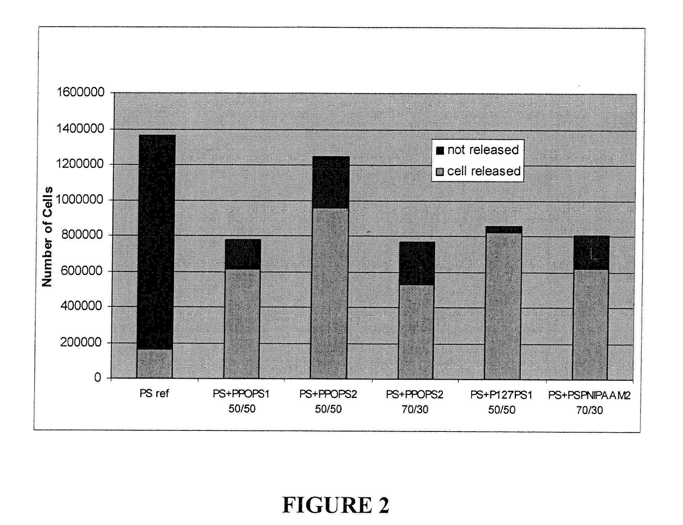

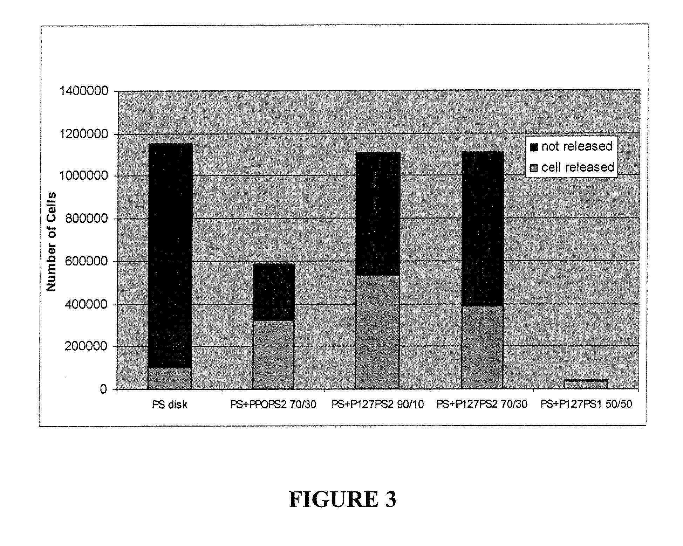

In the examples presented in Figures 2 and 3, the blends of polystyrene and block copolymers were prepared by mixing the polymers in anisole (20 wt% polymer content) and evaporating the solvent to obtain solid blends. The blends were then annealed at 150 °C under vacuum for 2 hours. Blends containing PPO-PS and P127-PS copolymers were transparent for thermo-responsive polymer content below 20 wt%. Other mixing techniques such as extrusion or injection molding could be used alternatively.

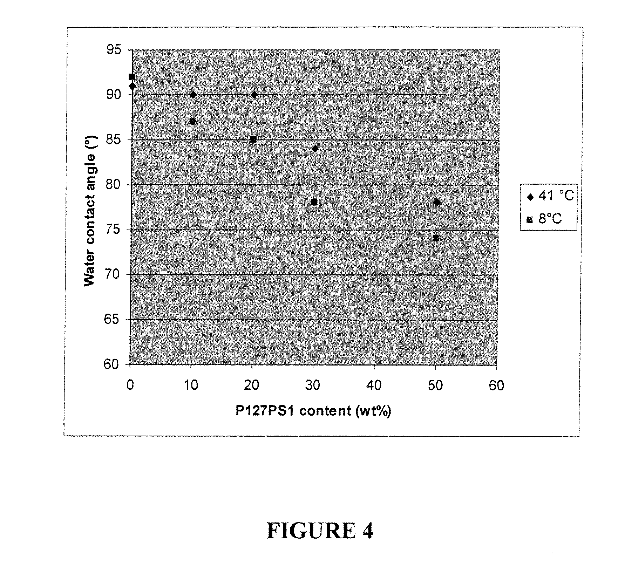

As described above, cell attachment or detachment is closely related to the hydrophobicity or hydrophilicity of the culture substrate. The hydrophobicity or hydrophilicity of a solid surface is characterized by the water contact angle. The hydrophilicity increases when the water contact angle decreases. The variation of the water contact angle on blends composed of polystyrene and PS127-PS1 thermo-responsive block copolymers is illustrated Figure 4. The water contact angle was measured at 2 different temperatures (above and below the LCST for different compositions of blends). The water contact angle measured for polystyrene/P127PS1 (LCST 27°C) blends exhibited a significant dependence with temperature, which indicates that the hydrophilicity of the substrate is temperature dependent. This dependence in temperature of the hydrophobicity of a few degrees in contact angle is sufficient to cause cell release. Differences of several degrees were also measured using other thermo-responsive blends. For a blend containing 20 wt% of PPOPS2 (LCST 10-15 °C), the contact angle was 84 ° at 22 °C and 77° at 7 °C. For a blend containing 30 wt% of PSPNIPAAM2 (LCST 32 °C), the contact angle was 95 ° at 41 °C and 91 ° at 20 °C.

Other mixing techniques such as extrusion can be used alternatively. A 35 mm large and 1.5 mm thick sample was prepared by extrusion using a DSM midi-extruder followed by hot pressing. Copolymers containing PNIPAAM, PPO and P127 segments were used in this method. The temperature during extrusion was between 140 °C and 170 °C depending on the viscosity of the blend. The temperature during pressing was between 130 °C and 150 °C.

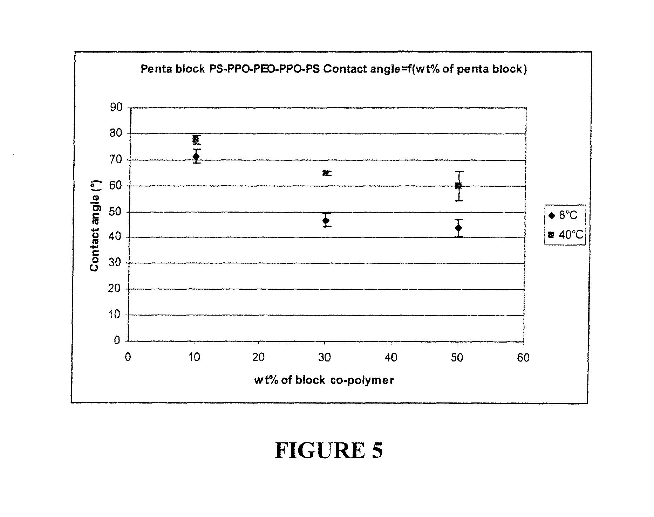

Blends prepared from extrusion/hot pressing exhibited more significant thermo-responsive behavior as shown in Figure 5. The difference in contact angle below and above the LCST, measured was between 6 and 20°. This stronger thermo-responsive behavior can be tentatively explained by an enrichment of the surface in low molecular weight thermo-responsive co-polymer due to the shear applied during extrusion.

### EXAMPLE 3-Cell Culturing using Blends Composed of Copolymers obtained via solution

Using the blends described above, prepared by evaporation of 20 wt% solutions of polymer in anisole, cell culturing and harvesting were studied using CHOβ3 cells. This cell line adheres to plastic substrates and therefore constitutes a good test for cell release. Using standard culture conditions adapted to this cell line, adhesion rate after 1h in a mixture of medium with 10% fetal bovine serum was between 48% for PS+PPOPS2 50/50 wt% (around 25 wt% PPO) and 73% for PS+PPOPS2 70/30 wt% (around 15 wt% PPO). In comparison, tissue culture treated polystyrene wells exhibited typically 80-85% adhesion. The total number of cells after thermal release and after trypsin treatment was between 56 and 91 % of the number counted on bare polystyrene (Figure 2). After lowering the temperature to 4 °C for 2 hours for PS, PPO-PS and P127-PS copolymers and to room temperature for PS-PNIPAAM, between 69% (PS+PPOPS2 70/30 wt%) and up to 96% (PS+P127-PS 50/50 wt%) of the total number of cells obtained after treatment with a trypsin solution were released, compared to 12% for the polystyrene substrate. Thus, the thermo-responsive copolymer has a significant effect on cell release. Treatment with a trypsin solution is unnecessary; therefore purification steps can be suppressed and the function of cells remains unaffected.

### EXAMPLE 4-Cell Culturing using Disks of Blends Composed of Copolymers obtained via extrusion and hot pressing

Using the blends described above prepared via extrusion and hot pressing, cell culturing and harvesting were studied using CHOβ3 cells. Using standard culture conditions adapted to this cell line, adhesion after 1h in a mixture of medium with 10% fetal bovine serum was 66% for PS+PPOPS2 70/30 wt%, 65% for PS+P127PS2 90/10 wt%, 61% for PS+P127PS2 70/30 wt%, and 55% for PS+P127PS2 50/50 wt%. By comparison, cell adhesion after 1h was 29% on a polystyrene disk prepared using the same process. The total number of cells obtained by addition of the number of cells counted after thermal release at 4°C for 2 hours and counted after trypsin treatment is reported in the Figure 3, as well as the ratio of cells released by thermal treatment. The overall number of cells counted was the same on bare polystyrene and on blends **PS+P127PS2** 90/10 wt% and PS+P127PS2 70/30 wt%. The total number collected on the blend PS+PPOPS2 70/30 wt% was almost half of the number collected on bare polystyrene. The number collected on the blend PS+P127PS1 50/50 wt% was only 4% of the number counted on bare polystyrene. The proportion of cells released after 2 hours at 4°C was 55% for PS+PPOPS2 70/30 wt%, 48% for PS+P127PS2 90/10 wt%, 35% for PS+P127PS2 70/30 wt%, and 85% for PS+P127PS 1 50/50 wt%. By comparison, the proportion of cells released at 4°C for 2hours was 9% on polystyrene. The thermo-responsive copolymer has therefore a significant effect on the release of cells below the LCST.

### EXAMPLE 5-Preparation of a thermo-responsive nanofibers mat deposited onto a Nylon woven mesh

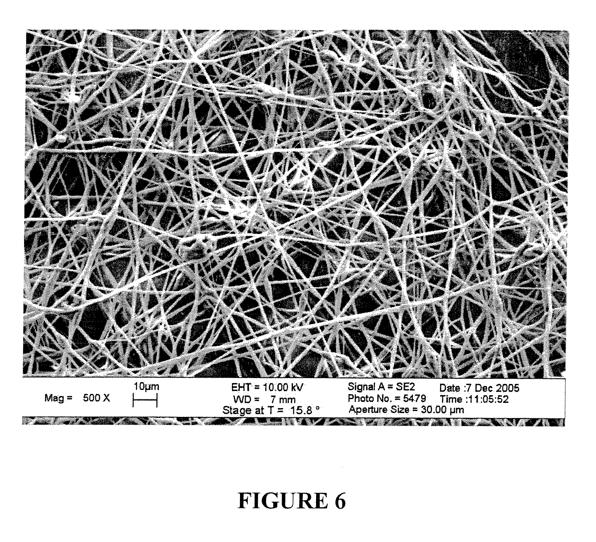

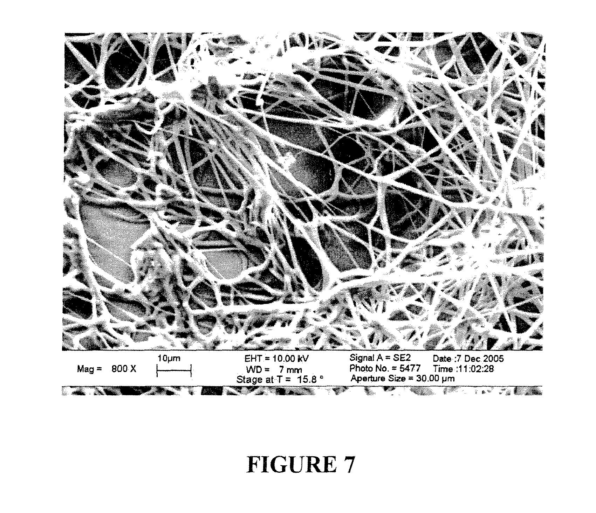

Poly(N-isopropylacrylamide) [25189-55-3] (0.75 g) having a average Mw of 300,000 and available from Scientific Polymer Products Inc. was dissolved in 17g of dimethylformamide (DMF) under stirring. In another vial, 14.25g of poly(methyl methacrylate) (PMMA) [9011-14-7] powder having an average Mw of 120,000 from Sigma Aldrich (ref #18,223-0) was dissolved in tetrahydrofuran (THF) under stirring and heating at 70 °C then cooled to room temperature. The solution of poly(N-isopropylacrylamide)/DMF solution was added to the PMMA/THF solution under stirring. The solution was clear and no phase separation was observed. The solution was loaded in a syringe equipped with a metal needle and electrospun by applying an electric field of about 2KV/cm between the needle and the grounded electrode of about 10 cm. The electrospun fibers were collected on a Nylon woven mesh applied on the top of the grounded electrode as temporary substrate. The SEM pictures in Figures 6 and 7 show the morphology of the fibrillar material obtained containing 5 wt % of thermo-responsive compound. The nanofibers have a diameter lower than 1 µm and are substantially free of beads. The dried nanofibers contain 5 wt% of thermo-responsive compound and 95% of hydrophobic polymer.

Throughout this application, various publications are referenced. The disclosures of these publications in their entireties are hereby incorporated by reference into this application in order to more fully describe the compounds, compositions and methods described herein.

Various modifications and variations can be made to the materials, methods, and articles described herein. Other aspects of the materials, methods, and articles described herein will be apparent from consideration of the specification and practice of the materials, methods, and articles disclosed herein. It is intended that the specification and examples be considered as exemplary.

## Claims

1. A thermo-responsive blend comprising at least one thermo-responsive polymer and at least one non-thermo-responsive/non-water soluble polymer.

2. The blend of claim 1, wherein the thermo-responsive polymer has a lower critical solution temperature or an upper critical solution temperature between 0 °C and 85 °C.

3. The blend of claim 1, wherein the thermo-responsive polymer comprises a homopolymer or copolymer of a (meth)acrylamide, an N-substituted acrylamide, an N,N-di-substituted (meth)acrylamide, an N-substituted (meth)acrylamide, an N,N-di-substituted (meth)acrylamide, a vinyl ether, an alkyl vinyl ether, an N-vinyl caprolactam, or a combination thereof.

4. The blend of claim 1, wherein the thermo-responsive polymer comprises a homopolymer or copolymer of acrylamide, methacrylamide, N-ethyl acrylamide, N-n-propyl acrylamide, N-n-propyl methacrylamide, N-isopropyl acrylamide, N-isopropyl methacrylamide, N-cyclopropyl acrylamide, N-cyclopropyl methacrylamide, N-ethoxyethyl acrylamide, N-ethoxyethyl methacrylamide, N-tetrahydrofurfuryl acrylamide, N-tetrahydrofurfuryl methacrylamide, N,N-dimethyl (meth)acrylamide, N,N-ethylmethyl acrylamide, N,N-diethyl acrylamide, 1-(1-oxo-2-propenyl)-pyrrolidine, 1-(1-oxo-2-propenyl)-piperidine, 4-(1-oxo-2-propenyl)-morpholine,1-(1-oxo-2-methyl-2-propenyl)-pyrrolidine, 1-(1-oxo-2 -methyl-2-propenyl)-piperidine, 4-(1-oxo-2-methyl-2-propenyl)-morpholine, methyl vinyl ether, or a combination thereof.

5. The blend of claim 1, wherein the thermo-responsive polymer comprises a homopolymer of N-isopropyl acrylamide.

6. The blend of claim 1, wherein the thermo-responsive polymer comprises polyethylene oxide, polypropylene oxide, a block copolymer of polyethylene oxide-polypropylene oxide, or a cellulose ether derivative.

7. The blend of claim 1, wherein the non-thermo-responsive/non-water soluble polymer comprises a polyester, a polyamide, a polycarbonate, a polycaprolactone, a polyurethane, a polyimide, a polyamide-imide, a poly-lactide-co-glycolide, a polyolefin, a cyclic polyolefin, a polysulfone, a poly (meth)acrylate polymer, a poly vinyl polymer, or a combination thereof.

8. The blend of claim 1, wherein the thermo-responsive polymer comprises a homopolymer of N-isopropyl acrylamide and the non-thermo-responsive/non-water soluble polymer comprises polystyrene.

9. The blend of claim 1, wherein the thermo-responsive polymer comprises at least one hydrophobic unit.

10. The blend of claim 9, wherein the hydrophobic unit is derived from H₂C=CHR, wherein R comprises an alkyl group, an aryl group, an alkoxy group, an amide group, or an ester group.

11. The blend of claim 9, wherein the hydrophobic unit is derived from styrene.

12. The blend of claim 1, wherein the thermo-responsive polymer comprises a copolymer of (1) N-isopropyl acrylamide, polyethylene oxide, a polypropylene oxide, or a block copolymer of polyethylene oxide-polypropylene oxide, or a combination thereof and (2) styrene.

13. The blend of claim 1, wherein the thermo-responsive polymer comprises a block copolymer of poly(prapylene oxide)-poly(ethylene oxide)-polystyrene, a block copolymer of poly(propylene oxide)-polystyrene, or a block copolymer of poly(N-isopropylacrylamide)-polystyrene.

14. A substrate for immobilizing cells or tissue, wherein the substrate comprises the blend of claim 1.

15. An article comprising the blend of claim 1.

16. A substrate for immobilizing cells or tissue, wherein the substrate comprises the blend of claim 9.

17. An article comprising the blend of claim 9.

18. A method for a plurality of cells, comprising (a) depositing a parent set of cells on the substrate of claim 14, and (b) the substrate with the deposited cells to promote the growth of the cells.

19. A method for growing a plurality of cells, comprising (a) depositing a parent set of cells on the substrate of claim 16, and (b) culturing the substrate with the deposited cells to promote the growth of the cells.

20. A method for differentiating cells, comprising (a) depositing a parent set of cells on the substrate of claim 14, and (b) culturing the substrate to promote differentiation of the cells.

21. A method for determining an interaction between a known cell line and a drug, comprising (a) depositing the known cell line on the substrate of claim 14; (b) contacting the deposited cells with the drug; and (c) identifying a response produced by the deposited cells upon contact with the drug.

22. A method for separating a compound present in a solution, comprising (a) contacting the substrate of claim 14 with the solution, wherein the compound is immobilized on the substrate; and (b) removing the immobilized compound from the substrate.

23. A method for growing tissue, comprising (a) depositing a parent set of cells that are a precursor to the tissue on the substrate of claim 14, and (b) culturing the substrate with the deposited cells to promote the growth of the tissue.

24. A nanofiber comprising the blend of claim 1.

25. A nanofiber comprising the blend of claim 9.

26. A substrate for immobilizing cells or tissue comprising:
a. a network of nanofibers, wherein the network of nanofibers comprises one or more blends of claim 1; and
b. a base substrate comprising a first outer surface, wherein the network of nanofibers is adjacent to the first outer surface of the base substrate.

27. A substrate for immobilizing cells or tissue comprising:
a. a network of nanofibers; and
b. a base substrate comprising a first outer surface, wherein the network of nanofibers is adjacent to the first outer surface of the base substrate, and
wherein the base substrate comprises one or more blends of claim 1.

28. A kit comprising (1) a network of nanofibers and (2) a base substrate, wherein the network of nanofibers, the base, or both the network of nanofibers and the base comprises one or more blends of claim 1.

29. A thermo-responsive polymer comprising at least one hydrophobic unit.
